# EUROPÄISCHE PATENTANMELDUNG

(11) **EP 1 870 062 A1**
(43) Veröffentlichungstag der Anmeldung: **26.12.2007**
(21) Anmeldenummer: 07011231.3
(22) Anmeldetag: 08.06.2007
(51) Int. Cl.: A61F 5/37

(54) **Schulterbandage**

(30) Priorität: 19.06.2006 DE 102006028385
(71) Anmelder: John GmbH, 99830 Treffurt (DE)
(72) Erfinder: John, Udo, 99988 Wendehausen (DE)
(74) Vertreter: Seckel, Uwe

(57) **Zusammenfassung**

Die Erfindung betrifft eine Schulterbandage (1) zur Ruhigstellung des Ober- und Unterarms mit einem schulterseitigen Tragegurt (5), der über Nacken und Brust zum Halten des angewinkelten Unterarms geführt und mit einem handseitig vorgesehenen Halteband (8), das über den Rücken geführt und über den Oberarm gelegt und auf dem Halteband festgelegt wird. Die Schulterbandage wird durch zwei asymetrisch oder symetrisch kreuzende Bänder (2,3) gebildet, die im gemeinsamen Kreuzungspunkt (4) miteinander unlösbar verbunden sind, wobei die Stirnseiten (13,14,13a,14a) der jeweils dem Kreuzungspunkt gegenüberliegenden gleich langen Bandabschnitte zu einer elliptischen Schlaufe verbunden sind, die einerseits eine Auflage auf den Schulterbereich und andererseits eine Einlage für den Ellenbogenbereich ausbilden und wobei sich an der Verbindungsnaht (26) der Schlaufe für den Schulterbereich der Tragegurt unlösbar anschließt und an der Schlaufe für den Ellenbogenbereich sich die Armanlage (6) anschließt, die durch eine Handfixierung (7) begrenzt ist, an die sich das Halteband unlösbar anschließt.

## Beschreibung

Die Erfindung betrifft eine Schulterbandage zur Ruhigstellung des Ober- und Unterarms mit einem schulterseitigen Tragegurt, der über Nacken und Brust zum Halten des angewinkelten Unterarms geführt und mit einem handseitig vorgesehenen Halteband, das über den Rücken geführt und über den Oberarm gelegt und auf dem Halteband festgelegt wird.

Bandagen die mit einer derartigen Wickelweise angelegt werden, finden beispielsweise bei Oberarmfrakturen und Schulterluxationen Verwendung und sind bereits in den verschiedensten Ausführungsformen bekannt.

Eine gattungsgemäße Bandage ist aus der EP 0 198 482 A1 bekannt. Diese Bandage besteht aus einem den Unterarm und Oberarm aufnehmenden Schlauchteil, das am schulterseitigen Ende mit dem einen Trageband und das am handseitigen Ende mit einem Halteband versehen ist, wobei beim Anlegen der Bandage das Trageband über Nacken und Brust zum Halten des angewinkelten Unterarms geführt wird und das handseitig vorgesehene Halteband über den Rücken geführt in einer Schlaufe über den Oberarm gelegt und rückläufig auf dem Haltegut fixiert wird.
Bei dieser Bandage ist infolge des geschlossenen einteiligen Schlauchteils, der über den Unterarm und Oberarm übergezogen wird, eine starke Schweißbildung unvermeidbar. Eine Schweißbildung unter dem Schlauchteil kann aber häufig zu einem heftigen Juckreiz führen und insbesondere in der Beuge des Ellenbogenbereichs zu schmerzhaften Entzündungen. Durch die geschlossene einteilige Ausführung des Schlauchteils ist aber auch das Anlegen der Bandage sowie das Abnehmen, beispielsweise zum Zweck der Reinigung der Verletzung oder des Arms erschwert. Aber auch die Herstellung der Bandage selbst ist durch die unterschiedlichen Materialien der einzusetzenden Teile kostenaufwendig.

Eine ähnliche Bandage wird auch in der DE 94 16 590 U1 offenbart. Bei dieser Bandage ist zusätzlich am Schlauchteil und zwar im Bereich des Unterarms eine veränderbare Führungslasche zur Aufnahme eines weiteren Zuggurts positioniert, um den zusätzlichen Zuggurt, der über die verletzte Schulter zum Unterarm geführt ist, am Unterarm lagestabil zu fixieren.

Aus der EP 0988 006 B1 ist eine gattungsgemäße Bandage bekannt, die ein halbschalenförmig ausgeformtes Oberarmteil mit einer kappenartigen Ausprägung zur Einbettung des Schultergelenks aufweist und aus einem halbschalenförmig ausgeformte Unterarmteil mit einer Einfassung des Ellenbogengelenks besteht, die über einen Gurt miteinander verbunden sind. Eine derartig ausgebildete Bandage weist sowohl im Schulter- und Oberarmbereich als auch im Unterarmbereich große Kontaktflächen zum Arm und Körper des Patienten auf, die ebenfalls eine verstärkte Schweißbildung nicht ausschließen. Aber auch die Herstellung der Bandage ist durch die halbschalig zu formenden Oberarm- und Unterarmteile sehr kostenaufwendig und damit teuer, so dass sich ihre Anwendung nur auf ganz bestimmte Sonderfälle beschränkt.

Aus der DE 40 29 622 C1 ist eine Bandage bekannt, die aus einem einteiligen im wesentlichen undehnbarem Band besteht, wobei das Band an seinen beiden Enden Befestigungsmittel zur Bildung einer Schlaufe oder Manschette aufweist und eine Länge hat, die es gestattet, das Band um den abgewinkelten Unterarm oder Handgelenk zu legen und über die der verletzten Schulter, dem verletzten Arm oder Oberarm gegenüberliegenden Seite geführt und von dort aus schräg über den Rücken und dann einmal in etwa Taillenhöhe um den Oberkörper herum bis zum Oberarm der betreffenden verletzten Körperseite und um diesen herum zu führen.
Diese Bandbandage ist zwar sehr wirtschaftlich herzustellen und vermeidet auch bei einer entsprechenden Materialart weitgehend die Schweißbildung und verfügt damit über verbesserte Trageeigenschaften für den Patienten, hat jedoch den wesentlichen Nachteil, dass keine ausreichende Ruhigstellung des Unterarms erreicht wird. Folglich wird das Gewicht des Unterarms zur Erzeugung eines im Bereich des Schultergelenks erforderlichen Niederhaltedrucks oder eine völlige Entlastung des Schulterbereichs nicht hinreichend genutzt. Aber auch das Anlegen dieser Bandbandage ist erschwert und bedarf einer großen Sorgfalt, da diese Bandage mehrmals über die Rückenpartie gelegt werden muss, was im liegenden Zustand für einen Patienten sehr unangenehm und schmerzhaft sein kann.

Diese und auch weitere bekannte Bandagen, insbesondere die, die ohne körpergerechte Ober- und Unterarmformteile ausgebildet sind, weisen bisher keine Auflageflächen auf, die sich allein durch die konstruktive Auslegung der Bandage formgerecht an die Konturen des menschlichen Körpers anpassen und in jeder Situation einen hohen Tragekomfort und eine stabile Lagefixierung der Bänder der Bandage sichern.

Aus der US 2003/01 35 141 A1 ist eine Schulterbandage bekannt geworden, die aus zwei sich asymmetrisch unter einem Winkel kreuzenden Bändern besteht, die einerseits an einem anatomisch der Schulter angepassten einstückigen Schulterpolster und andererseits an einer Unterarmschlaufe festgelegt sind, so dass sich elliptische Schlaufen sowohl zur Auflage im Schulterbereich als auch für die Einlage des Ellenbogenbereichs ergeben, wobei am Schulterpolster sich ein Tragegurt anschließt, der über den Nacken und anschließend brustseitig verläuft und den angewinkelten Unterarm im Bereich des Handgelenks mit einer Schlaufe umschließt, und an der Unterarmschlaufe eine Einlage des Ellenbogenbereichs unlösbar angebracht ist. Die Handfixierung, die im Bereich des Handgelenks den Unterarm umschließt, ist an einem zusätzlichen Halteband fest verbunden, das über den Rücken und in einer Schlaufe über den Oberarm geführt ist und rückläufig mit seinem freien Ende auf dem Halteband festgelegt ist.

Aus der DE 197 45 705 C1 ist eine Schulterbandage bekannt, die aus einem getrennten gefertigten Oberarmteil und Unterarmteil besteht. Dabei ist das Oberarmteil U-förmig ausgebildet, wobei der in dem Bereich des Oberarmteils, in dem der erste und zweite Schenkel zusammenlaufen, liegende Mund auf der Schulter aufliegt, der erste Schenkel sich anterior und der zweite Schenkel sich posterior bis annähernd zum Ellenbogengelenk erstreckt, und das Unterarmteil aus drei Abschnitten gebildet ist, nämlich einem ersten Abschnitt, der bei in einem Winkel von ungefähr 90° anterior angewinkelten Unterarm im ularen Unterarmbereich liegt, einem zweiten Abschnitt, der mit dem zweiten Schenkel des Oberarmteils verbunden ist und der anterior über das Ellenbogengelenk verläuft und in den ersten Abschnitt mündet, einem dritten Abschnitt, der mit dem ersten Schenkel des Oberarmteils verbunden ist und posterior über das Ellenbogengelenk verläuft und in den ersten Abschnitt mündet, wobei die Enden des zweiten und des dritten Abschnitts miteinander verbunden sind und wobei an dem Oberarmteil ein Tragegurt und am Unterarmteil ein Haltegurt angebracht sind und wobei das Oberarmteil mit dem Tragegurt, der von der Schulter zum anterialen Thoraxbereich verläuft, nach ventral zum Unterarm im Bereich des Handgelenks fixiert wird und wobei der Haltegurt vom Unterarmteil von der Handregion kommend nach dorsal im Lendenbereich zum distalen Oberarm verläuft und in diesen von posterior nach anterior verlaufend nach lateral umschließt.

Bei beiden Ausführungsformen sind für die Schulterauflage und für die Aufnahme des Ellenbogenbereichs bzw. des Unterarms spezielle Formteile notwendig, die durch Ausschneiden von Zuschnitten aus flächigem Material vorgefertigt werden müssen. Zuschnitte führen aber zu einem zusätzlichen Fertigungs- und erhöhten Materialaufwand, der infolge von Zuschnittverlusten unvermeidbar ist. Die Herstellung von Bandagen mit Formteilen erfordert auch, dass für jede Größe von Schulterbandagen unterschiedlich vorgefertigte Größen von Formteilen notwendig sind, um zu gewährleisten, dass eine angelegte Bandage annähernd anatomisch an den Körperbau einer Patientengruppe angepasst ist, um auf diese Weise für die Dauer des Heilungsprozesses annähernd sicher zu stellen, dass form- und kraftschlüssig von extern auf den menschlichen Körper eingewirkt werden kann und gleichzeitig ein stabiler Sitz und ein schmerz- und behinderungsfreier Tragekomfort gegeben ist.

Durch die Formteile, die im allgemeinen in bestimmten Grenzbereichen abgestuft sind, kann aber auch nicht bei jedem Patienten sicher gestellt werden, dass die Auflagefläche im Schulterbereich, die Einlagefläche im Ellenbogenbogenbereich aber auch die Anlagenflächen am Körper nach dem Anlegen der Schulterbandage sich eindeutig anatomisch an den Körpers anpassen. Folglich ist nicht in jedem Fall gewährleistet, dass die zu übertragenden Kräfte in den menschlichen Körper großflächig verteilt eingeleitet werden, so dass der Tragekomfort und die Passformeffizienz und damit die Sitzqualität der Schulterbandage von Fall zu Fall mehr oder weniger beeinträchtigt ist.

Aufgabe der vorliegenden Erfindung ist es daher, eine Schulterbandage zur Verfügung zu stellen, die kostengünstig herstellbar ist, deren Auflageflächen sich nach dem Anlegen an die Konturen des menschlichen Körpers anpassen, die in jeder Situation am Körper lagefixiert ist und einen hohen Tragekomfort aufweist und die problemlos auch im narkotisierten Zustand am Patienten angelegt werden kann.

Diese Aufgabe wird erfindungsgemäß mit einer Schulterbandage mit den Merkmalen des Anspruches 1 gelöst. Die Schulterbandage besteht demgemäß aus zwei sich asymmetrisch oder symmetrisch in einem Winkel von 90° kreuzenden Bändern, die im gemeinsamen Kreuzungspunkt unlösbar oder lösbar miteinander verbunden sind, die jeweils die an ihren gegenüber dem Kreuzungspunkt liegenden Stirnseiten durch eine Verbindungsnaht miteinander unlösbar verbunden sind, so dass sich elliptische Schlaufen sowohl zur Auflage auf den Schulterbereich als auch für die Einlage des Ellenbogenbereichs ergeben und wobei sich an die Verbindungsnaht der Schlaufe des Schulterbereichs der Tragegurt anschließt und sich an die Schlaufe zur Einlage des Ellenbogenbereichs eine Unterarmauflage anschließt, die durch mindestens ein Band gebildet wird, das mit der Schlaufe zur Einlage des Ellenbogenbereichs unlösbar verbunden ist und eine Handfixierung aufweist, die mit dem Halteband fest verbunden ist.
Eine derartig ausgebildete Schulterbandage kann sehr wirtschaftlich und formteilfrei aus Bandabschnitten gleicher Breite und Qualität hergestellt und dem Verbraucher zu einem geringen Preis zur Verfügung gestellt werden.
Durch die Verbindung der sich in einem gemeinsamen Kreuzungspunkt asymmetrisch oder symmetrisch kreuzenden Bänder und der stirnseitigen Verbindung der beiden Bandabschnitte, die gemeinsam auf einer Seite des Kreuzungspunktes liegen, werden beiderseits des Kreuzungspunktes zwei elliptisch verlaufende und unterschiedlich lange oder gleichlange schalenähnliche Schlaufen gebildet, die auf der einen Seite des Kreuzungspunktes eine Schulterauflage und auf der Seite eine Ellenbogeneinlage bilden. Diese sich kreuzenden Bänder gehen von beiderseits der Schulter aus und kreuzen sich oberhalb des Ellenbogengelenkes. Nach dem Kreuzungspunkt verläuft das zuvor posterior verlaufende Band anterior, während das anterior verlaufende Band das Ellenbogengelenk posterior umschließt, so dass das Ellenbogengelenk von den beiden hinter dem Kreuzungspunkt liegenden Bandenden umschlossen ist und durch eine von beiden Bandenden umschlossene Öffnung hindurchragt. Damit wird eine wesentliche verbesserte und stabilere Bandlage sowohl im Schulter- und Ellenbogenbereich als auch dem Oberarm erreicht.
Durch die beiderseits des Kreuzungspunktes schalenartig verlaufenden Schlaufen passen sich die Auflagefläche im Schulterbereich und die Einlagefläche im Ellenbogenbereich aber auch die Anlageflächen am Körper bereits schon beim Anlegen und Befestigen der Schulterbandage eindeutig an die Konturen des Körpers an und leiten damit großflächig die zu übertragenden Kräfte in den menschlichen Körper ein.

Folglich wird durch großflächige Auf- und Anlage der Bänder und die damit erzwungene großflächige Einleitung der zu übertragenden Kräfte der Tragekomfort und auch die Sitzqualität der Schulterbandage wesentlich erhöht. Aber auch das Anlegen und das Lösen einer derartig ausgebildeten Schulterbandage kann sehr problemlos mit wenigen Handgriffen durchgeführt werden.

Mit dem stabilen Schlaufenverbund, der sowohl die Schulterauflage als auch die Einlage für den Ellenbogen ausbildet, kann beim Anlegen der Schulterbandage der Unterarm im Ellenbogenbereich bereits in einer angewinkelten Stellung so fixiert und ruhig gestellt werden, dass gesichert ist, dass das Gewicht des Unterarms zur Erzeugung eines im Bereich des Schultergelenks erforderlichen Niederhaltedrucks zur Verfügung steht.
Mit der eindeutigen Fixierung des Ellenbogenbereichs in der Schlaufe für die Einlage des Ellenbogenbereichs, die auf der gegenüberliegenden Seite des Kreuzungspunktes von der Auflage im Schulterbereich liegt, wird bereits der Unterarm in Verbindung mit der umschlingenden Schlaufe des Tragegurts, der brust- und nackenseitig verläuft und mit der Schulterauflage fest verbunden ist, in der angewinkelten Stellung ruhig gestellt. Folglich ist die Unterarmauflage für die eigentliche Ruhigstellung des Unterarms annähernd bedeutungslos und kann sehr vereinfacht nur als Distanzband zwischen dem Ellenbogenbereich und der Handfixierung ausgelegt werden. Da die Unterarmauflage im wesentlichen nur noch als Distanzband erforderlich ist, ist auch die Voraussetzung gegeben, dass die Unterarmauflage mit der Handfixierung und des daran angeschlossenen Haltebands lösbar mit einem zugfesten Befestigungsmittel an der Schlaufe zur Einlage des Ellenbogenbereichs austauschbar befestigt werden kann. Damit kann problemlos die Länge der Unterarmauflage durch einen Austausch so an den Patienten angepasst werden, dass der Unterarm eindeutig zwischen der Handfixierung und der Schlaufe für die Aufnahme des Ellenbogenbereichs fixiert ist.

Durch die offene konstruktive Ausbildung dieser Schulterbandage ist auch zwischen den Abschnitten der Bänder, die mit dem menschlichen Körper in Kontakt kommen, eine hinreichende Luftzirkulation möglich, so dass im Wesentlichen eine Schweißbildung im Kontaktbereich der Bänder mit dem Körper verhindert werden kann.

Vorteilhafterweise ist beiderseits des Kreuzungspunktes ein Zugstück vorgesehen, das mit der Schlaufe zur Auflage im Schulterbereich im unteren Abschnitt und mit der Schlaufe zur Einlage des Ellenbogenbereichs im oberen Abschnitt unlösbar verbunden ist. Mit diesen Zugstücken kann ein wesentlicher Teil der innerhalb der Bänder wirkenden Kräfte unmittelbar auf den gekreuzten Teil des jeweils anderen Bandes übertragen werden, so dass durch diese Zugstücke der Kreuzungspunkt erheblich entlastet wird und somit, wenn gewünscht, auch lösbar ausgebildet sein kann. Auf jeden Fall kann damit der Sitz der gekreuzten Bänder sowohl im Schulterbereich als auch im Ellenbogenbereich noch weiter verbessert werden.

Diese Zugstücke können auch aus einem elastischen Material bestehen, um damit die Voraussetzung zu schaffen, dass die Abschnitte der Bänder sowohl im Schulterbereich als auch im Ellenbogenbereich sich auch dann an die Körperkonturen anpassen, wenn beispielsweise der Brustbereich im Schulterabschnitt stärker ausgebildet ist als die dazu korrespondierende Rückenpartie.

Die Unterarmauflage kann auch aus mindestens zwei Bändern bestehen, die zueinander beabstandet und mit der Schlaufe zur Aufnahme des Ellenbogenbereichs und mit der Handfixierung unlösbar oder lösbar verbunden sind. Mit einer lösbaren Befestigung der Bänder ist die Voraussetzung gegeben, dass die Länge der Unterarmauflage variiert werden kann und damit exakt an die Unterarmlänge so angepasst werden kann, dass die Handfixierung mit dem Halteband in dem Bereich liegt, in dem der Unterarm zur Schlaufe der Ellenbogenaufnahme eindeutig lagefixiert ist. Wenn gewünscht, kann auch zwischen den Bändern der Unterarmauflage ein unlösbares oder lösbares gazeartiges Teil eingelegt werden.

Die Stirnseiten der sich kreuzenden Bänder sind bevorzugt mit einem Winkel versehen, bei dem die Auflageflächen der Bänder nach dem Verbinden der Stirnseiten sich so aus einer Ebene erheben und räumlich an die Konturen des menschlichen Körpers anpassende Aufnahmeflächen bilden. Damit sind die Auflageflächen und Anlageflächen der Schulterbandage bereits vorpositioniert und nehmen bereits in jedem Größenbereich der Schulterbandage beim Anlegen eine Lage ein, die dem Körperbau des Patienten sehr nahe kommt.

Der mit der Schulterauflage verbundene Trägergurt, der über den Nacken verläuft und brustseitig zum angewinkelten Unterarm geführt wird, ist um den Unterarm mit einer geschlossenen Schlaufe oder lösbaren Schlaufe gelegt. Als bevorzugte Form sollte jedoch die an sich bekannte lösbare Schlaufenbildung gewählt werden, bei der das Ende des Trägergurts nach dem Umschlingen des Unterarms mittels eines Befestigungsmittels auf den Trägergurt festgelegt ist, um den Trägergurt so an den Körper anzupassen, dass der Unterarm immer annähernd im rechten Winkel steht.

Um den Tragekomfort der Schulterbandage weiter zu verbessern und um die Voraussetzung zu schaffen, dass die lösbaren Verbindungen einfach ausgelegt werden können, bestehen alle Bänder aus einem hautfreundlichen, atmungsaktiven und klettbaren Material und/oder die Bänder sind einschichtige oder vernähte oder kaschierte Doppelbänder, die einen Zwischenraum ausbilden, in dem mindestens teilweise ein atmungsaktives Material als Polsterschicht oder Stabilisierungsschicht fixiert eingelegt ist.

Vorteilhafterweise sind die Bänder mit einer mittig verlaufenden Stabilisierungsnaht versehen, die bevorzugt eine Mehrstich-Zickzack-Naht ist. Damit erhalten die Bänder der in Bandform hergestellten Schulterbandage eine ausreichende Formstabilität und das Anlegen der Schulterbandage ist auch nach mehrmaligem Gebrauch problemlos möglich.

Die unlösbaren Verbindungen zwischen den einzelnen Bandagenabschnitten und des Kreuzungspunktes, der allseitig verbunden ist, bestehen vorteilhafterweise aus Nähverbindungen, die bevorzugt mit einer Mehrstich-Zickzack-Naht ausgeführt werden. Selbstverständlich sind auch andere unlösbare Verbindungsarten, wie beispielsweise Kleben, Schweißen u.s.w. anwendbar.

Die genutzten lösbaren Verbindungen sind als Klettverbindungen ausgeführt, die in Verbindung mit der vorgeschlagenen Materialart der Bänder die schnellste, variabelste und wirtschaftlichste Verbindungsform ist. Auch hier sind selbstverständlich andere lösbare Verbindungsformen anwendbar, wie beispielsweise Druckknöpfe, Hakenverbindungen u.s.w..

Die Schulterbandage kann auch mindestens teilweise einstückig aus einem Gestrick oder Gewirk bestehen. Damit besteht die Möglichkeit die Herstellungskosten weiter zu senken und dem Verbraucher noch kostengünstigere Schulterbandagen zur Verfügung zu stellen.

Nach einer weiteren besonderen Ausführungsform der Erfindung wird vorgeschlagen, dass nach dem Anlegen der Schlaufe für den Schulterbereich der Oberarm und die Schlaufe von einem zusätzlichen oder mit der Schlaufe verbundenen und lösbaren Band umschlossen ist, auf dem das Halteband festgelegt ist, nach dem es zwischen dem Körper und dem Oberarm durchgeführt ist oder dass das Halteband durch eine körperseitig an dem zusätzlichen Band unlösbare vorgesehene Schleife gezogen wird und anschließend rückläufig auf dem Halteband fixiert ist.

Diese Wickelweise, die von der allgemein bekannten Wickelweise für Bandagen geringfügig abweicht, empfiehlt sich aber besonders bei Kleinstkindern und bei Patienten, die auf Grund einer psychischen Erkrankung unbewusste und unkontrollierte Bewegungsabläufe vollziehen und daher angenommen werden kann, dass die an sich sichere und stabile Befestigung der Schulterbandage mit der allgemein bekannten Wickelweise der Bandage keine dauerhafte Ruhigstellung des Ober- und Unterarms gewährleistet.

Weitere Einzelheiten der Erfindung ergeben sich aus der nachfolgenden ausführlichen Beschreibung und der beigefügten Zeichnungen, in denen bevorzugte Ausführungsformen der Erfindung gezeigt sind.

In den Zeichnungen zeigen:
- Fig. 1:: eine schematische Seitenansicht der Schulterbandage im angelegten Zustand,
- Fig. 2:: eine schematische Darstellung der Grundform der gekreuzten Bänder,
- Fig. 3:: eine schematische Darstellung einer Schulterbandage,
- Fig. 4:: eine schematische Darstellung einer weiteren Ausführungsform einer Schulterbandage,
- Fig. 5:: eine schematische Darstellung des Zusatzbands mit Verlauf des Haltegurts.

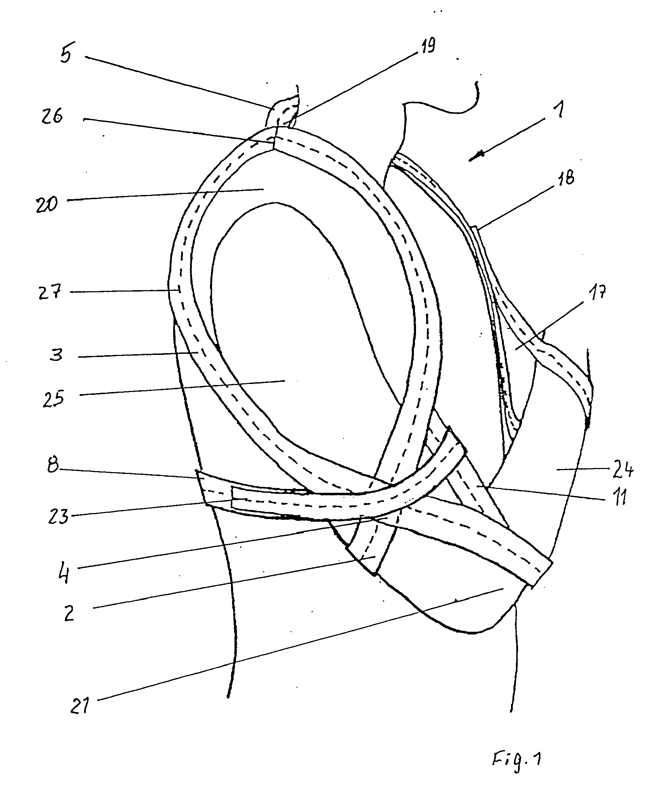

Die in Fig. 1 gezeigte angelegte Schulterbandage 1 besteht im wesentlichen aus den Bändern 2, 3, einem Tragegurt 5, einem Halteband 8, einer Unterarmauflage 6, einer Handfixierung 7, die bevorzugt aus einem hautfreundlichen, atmungsaktiven und klettbaren Material bestehen.

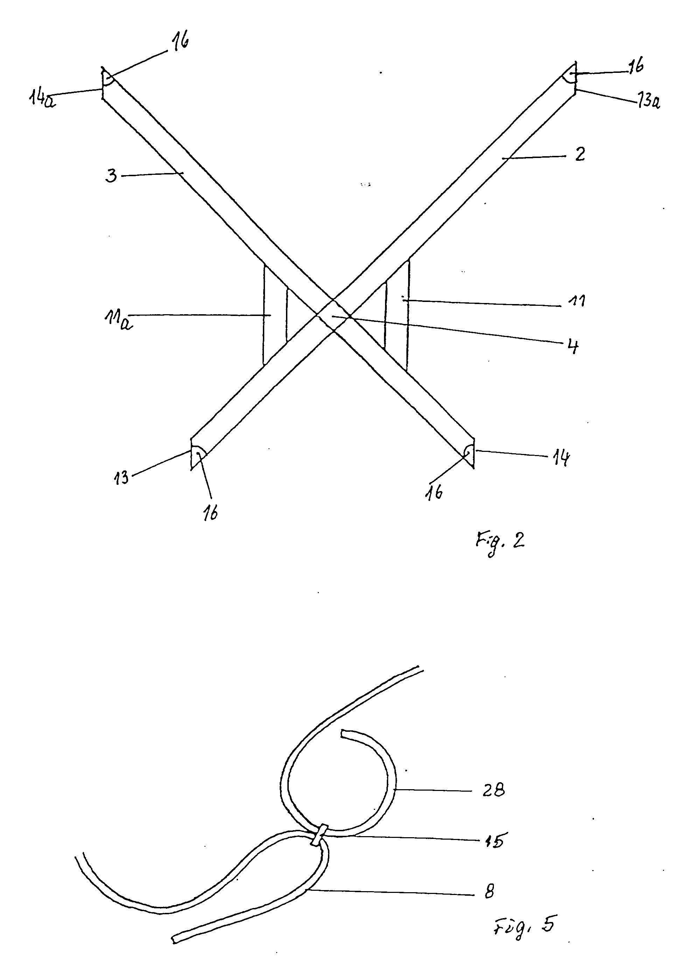

Wie in Fig. 2 gezeigt, wird die Schulterbandage 1 aus zwei sich einander bevorzugt asymmetrisch in einem Winkel von 90° kreuzenden Bändern 2, 3 gleicher Breite gebildet, die im gemeinsamen Kreuzungspunkt 4 vorteilhafterweise mit einer nicht weiter gezeigten Nähverbindung unlösbar miteinander verbunden sind.

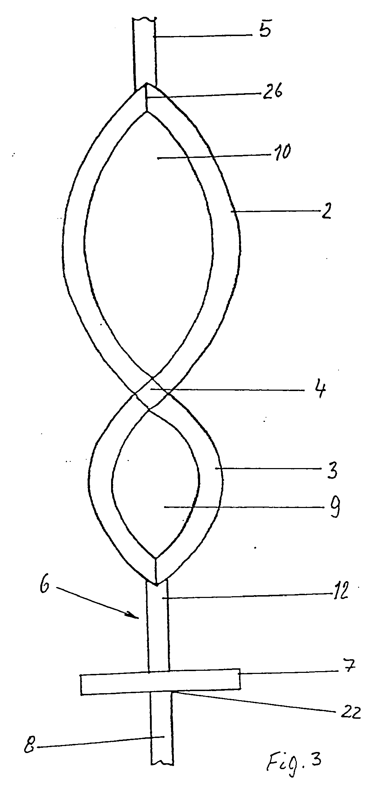

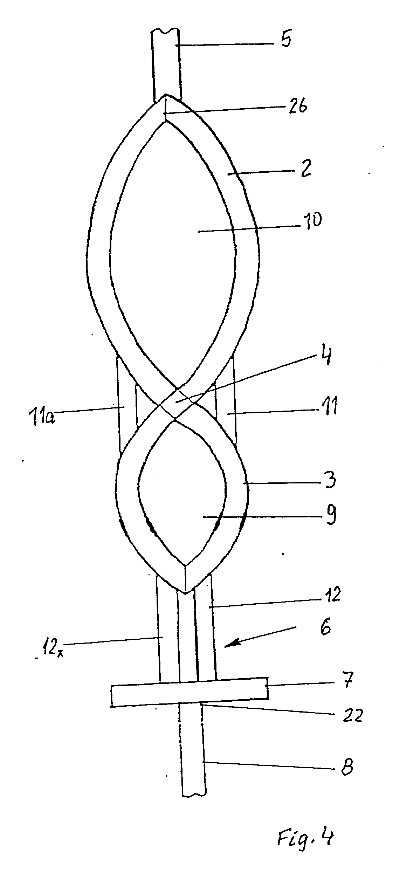

Die gegenüber dem Kreuzungspunkt 4 liegenden Bandabschnitte der Bänder 2, 3, weisen jeweils eine gleiche Länge auf und sind an ihren Stirnseiten 13, 14 bzw. 13a, 14a in einem Winkel 16 geschnitten, der bevorzugt 30° - 60°, in der gezeigten Ausführungsform 45°, beträgt. Die Stirnseiten 13, 14 bzw. 13a, 14a sind durch eine Nähverbindung unlösbar verbunden und bilden zwei ellipsenförmig verlaufende Schlaufen 9, 10 aus, die auf einer gemeinsamen Ebene dem Kreuzungspunkt 4 gegenüberliegen, wie in den Fig. 3, 4 gezeigt, wobei die größere Schlaufe 10 die Auflagefläche für den Schulterbereich 20 und die kleinere Schlaufe 9 die Einlagefläche für den Ellenbogenbereich 21 bildet.

Mittig auf der Verbindungsnaht 26 der Schlaufe 10 für den Schulterbereich 20 ist ein Tragegurt 5 mit dem einen Ende 19 fest mit der Schlaufe 10 verbunden. Der Tragegurt 5 ist dabei in einer Länge ausgelegt, die geeignet ist, den Tragegurt 5 über den Nacken und den Brustbereich bis zum angewinkelten Unterarm 24 zu führen und gewährleistet, dass nach dem Umlegen um den Unterarm 24 eine Halteschlaufe gebildet werden kann, in dem das freie Ende 18 des Tragegurts 5 auf dem brustseitig verlaufenden Abschnitt des Tragegurts 5 bevorzugt mittels einer Klettverbindung festgelegt wird.

Unmittelbar hinter der Schlaufe 9 für die Einlage des Ellenbogens schließt sich in Richtung auf die Hand eine Unterarmauflage 6 an, die bevorzugt aus zwei einander parallel verlaufende Bänder 12, 12x besteht, die an der Schlaufe 9 mit einer zugbelastbaren Verbindung unlösbar oder lösbar festgelegt sind und zwischen sich einen parallel verlaufenden Freiraum ausbilden.

Die Unterarmauflage 6 kann aber auch aus einem oder mehrfach parallel verlaufenden Bändern 12; 12x gebildet werden, wobei bei zwei oder mehrfach vorgesehenen Bändern 12, 12x der jeweils parallel verlaufende Zwischenraum durch ein nicht gezeigtes gazeartiges Teil unlösbar oder lösbar überdeckt sein kann.

Die aus den Bändern 12, 12x bestehende Unterarmauflage 6 mündet in einer Schlinge, die unlösbar mit den Bändern 12, 12x verbunden ist und sich quer zu den beiden Bändern 12, 12x erstreckt und eine das Handgelenk umschließende Schlinge als Handfixierung 7 ausgebildet, die bevorzugt mit Hilfe eines Klettverschlusses so eingestellt werden kann, das das Handgelenk fest umschlossen ist.

Die nicht näher gezeigte Handfixierung 7 erfolgt bevorzugt durch das quer zu der Unterarmauflage 6 verlaufende Band, in der Weise, dass mit dem Band hinter dem Daumen eine Schlaufe gebildet wird, die ebenfalls bevorzugt durch eine Klettverbindung lösbar geschlossen wird.

An der Handfixierung 7 oder auch dem Band der Unterarmauflage 6, in diesem Fall ist die Handfixierung 7 auf dem Band der Unterarmauflage 6 unlösbar befestigt, schließt sich unmittelbar ein Halteband 8 unlösbar mit seinem einen Ende 22 an. Das Halteband 8 weist dabei eine Länge auf, die geeignet ist, dass das Halteband 8 annähernd in Höhe der Taille um den Körper geführt und zwischen dem Oberarm 25 und Körper hindurch, um den Oberarm 25 herum und rückläufig mit dem anderen Ende 23 auf dem Halteband im Rückenbereich fixierbar ist.

Beiderseits des Kreuzungspunktes 4 der Bänder 2, 3 sind, wie in Fig. 2 und 4 gezeigt, zwei parallel verlaufende Zugstücke 11, 11a vorgesehen, die mit dem einen Ende an der Außenseite am unteren Bereich der Schlaufe 10 und mit dem anderen Ende an der Außenseite am oberen Bereich der Schlaufe 9 unlösbar befestigt sind. Diese Zugstücke 11, 11a können dabei aus dem gleichen Bandmaterial aber auch aus einem elastischen Material bestehen.

Mit diesen Zugstücken 11, 11a wird ein wesentlicher Teil der innerhalb der Bänder 2, 3 wirkenden Kräfte unmittelbar auf den kreuzenden Teil des jeweils anderen Bandes 2; 3 übertragen, so dass durch diese Zugstücke 11, 11a der Kreuzungspunkt 4 erheblich entlastet wird. Auf diese Weise wird der Sitz der Bänder 2, 3 sowohl im Schulterbereich 20 als auch im Ellenbogenbereich 21 erheblich verbessert.

Für eine abweichende Wickelweise der Schulterbandage 1 wird ein zusätzliches Band 28 vorgeschlagen, das auf einem Bandabschnitt des Bandes 2; 3 der Schlaufe 10 lösbar oder unlösbar festgelegt ist, auf dem das Halteband 8 mit dem Ende 23 fixierbar ist oder das mit einer zusätzlichen unlösbaren Schlaufe 15 versehen ist, durch die das Halteband 8 durchgeführt wird und rückläufig auf dem Halteband 8 fixiert ist.

Bevorzugt sind alle Bänder mittig mit einer Stabilierungsnaht 27 versehen, die bevorzugt eine Mehrstich-Zickzack-Naht ist.

Als lösbare Verbindung werden bis auf die zugstabile Verbindung einer lösbaren Unterarmauflage 6 an die Schlaufe 9 zur Aufnahme des Ellenbogenbereichs 21 bevorzugt Klettverbindungen genutzt. Ebenfalls werden für alle unlösbaren Verbindungen bevorzugt Nähverbindungen verwendet, die vorteilhafterweise als Mehrstich-Zickzack-Naht ausgeführt werden.

Als Wickelweise zum Anlegen der erfinderischen Schulterbandage 1 wird die im Stand der Technik beschriebene Wickelweise genutzt, die in den Fachkreisen allgemein bekannt ist.

### Aufstellung der Bezugszeichen

- 1: Schulterbandage
- 2: Band
- 3: Band
- 4: Kreuzungspunkt
- 5: Tragegurt
- 6: Unterarmauflage
- 7: Handfixierung
- 8: Halteband
- 9: Schlaufe - Ellenbogenbereich
- 10: Schlaufe - Schulterbereich
- 11: Zugstück
- 11a: Zugstück
- 12: Band
- 12x: Band
- 13: Stirnseite
- 13a: Stirnseite
- 14: Stirnseite
- 14a: Stirnseite
- 15: Schlaufe
- 16: Winkel
- 17: Halteschlaufe
- 18: Ende Tragegurt
- 19: Ende Tragegurt
- 20: Schulterbereich
- 21: Ellenbogenbereich
- 22: Ende Halteband
- 23: Ende Halteband
- 24: Unterarm
- 25: Oberarm
- 26: Verbindungsnaht
- 27: Stabilisierungsnaht
- 28: zusätzliches Band

## Patentansprüche

1. Schulterbandage zur Ruhigstellung des Ober- und Unterarms mit einem schulterseitigen Tragegurt (5), der über Nacken und Brust zum Halten des angewinkelten Unterarms (24) geführt und mit einem handseitig vorgesehenen Halteband (8), das über den Rücken geführt und über den Oberarm (25) gelegt und auf dem Halteband (8) festgelegt wird, wobei zwei sich asymmetrisch oder symmetrisch in einem Winkel von 90° kreuzende Bänder (2, 3) im gemeinsamen Kreuzungspunkt (4) unlösbar oder lösbar miteinander verbunden sind, die jeweils an ihren gegenüber dem Kreuzungspunkt (4) liegenden Stirnseiten (13, 14; 13a, 14a) durch eine Verbindungsnaht (26) miteinander unlösbar verbunden sind, so dass sich elliptische Schlaufen (9, 10) sowohl zur Auflage auf den Schulterbereich (20) als auch für die Einlage des Ellenbogenbereichs (21) ergeben und wobei sich an die Verbindungsnaht (26) der Schlaufe (10) des Schulterbereichs (20) der Tragegurt (5) anschließt und sich an die Schlaufe (9) zur Einlage des Ellenbogenbereichs (21) eine Unterarmauflage (6) anschließt, die durch mindestens ein Band (12) gebildet wird, das mit der Schlaufe (9) zur Einlage des Ellenbogenbereichs (21) unlösbar oder lösbar verbunden ist und eine Handfixierung (7) aufweist, die mit dem Halteband (8) fest verbunden ist.

2. Schulterbandage nach Anspruch 1, **dadurch gekennzeichnet, dass** beiderseits des Kreuzungspunktes (4) ein Zugstück (11; 11a) vorgesehen ist, das mit der Schlaufe (10) zur Auflage im Schulterbereich (20) im unteren Abschnitt und mit der Schlaufe (9) zur Einlage des Ellenbogenbereichs (21) im oberen Abschnitt unlösbar verbunden ist.

3. Schulterbandage nach Anspruch 2, **dadurch gekennzeichnet, dass** die Zugstücke (11, 11a) aus einem elastischen Material bestehen.

4. Schulterbandage nach einem der Ansprüche 1 bis 3, **dadurch gekennzeichnet, dass** die Unterarmauflage (6) aus mindestens zwei Bändern (12, 12x) besteht, die zueinander beabstandet mit der Schlaufe (9) zur Aufnahme des Ellenbogenbereichs (21) und mit der Handfixierung (7) unlösbar oder lösbar verbunden sind.

5. Schulterbandage nach einem der Ansprüche 1 bis 4, **dadurch gekennzeichnet, dass** die Stirnseiten (13, 13a, 14, 14a) der sich kreuzenden Bänder (2, 3) mit einem gleichen Winkel (16) versehen sind, bei dem die Auflageflächen der Bänder (2, 3) nach dem Verbinden der Stirnseiten (13, 14; 13a, 14a) sich so aus einer Ebene erheben und räumlich an die Konturen des menschlichen Körpers anpassende Aufnahmeflächen bilden.

6. Schulterbandage nach einem der Ansprüche 1 bis 5, **dadurch gekennzeichnet, dass** der Tragegurt (5) in einer geschlossenen oder lösbaren Halteschlaufe (17) um den Unterarm (24) gelegt ist.

7. Schulterbandage nach einem der Ansprüche 1 bis 6, **dadurch gekennzeichnet, dass** die Bänder (2, 3, 12, 12x), der Tragegurt (5) und das Halteband (8) eine gleiche Breite aufweisen.

8. Schulterbandage nach einem der Ansprüche 1 bis 7, **dadurch gekennzeichnet, dass** die Bänder (2, 3, 12, 12x), der Tragegurt (5) und das Halteband (8) aus einem hautfreundlichen, atmungsaktiven und klettbaren Material bestehen und/oder die Bänder einschichtige oder vernähte oder kaschierte Doppelbänder sind, die einen Zwischenraum ausbilden, in dem mindestens teilweise ein atmungsaktives Material als Polsterschicht oder Stabilisierungsschicht fixiert eingelegt ist.

9. Schulterbandage nach einem der Ansprüche 1 bis 7, **dadurch gekennzeichnet, dass** die Bänder (2, 3, 12, 12x), der Tragegurt (5) und das Halteband (8) mit einer mittig verlaufenden Stabilisierungsnaht (27) versehen sind.

10. Schulterbandage nach Anspruch 9, **dadurch gekennzeichnet, dass** die Stabilisierungsnaht (27) eine Mehrstich-Zickzack-Naht ist.

11. Schulterbandage nach einem der Ansprüche 1 bis 10, **dadurch gekennzeichnet, dass** die unlösbaren Verbindungen Nähverbindungen sind.

12. Schulterbandage nach Anspruch 11, **dadurch gekennzeichnet, dass** die Nähverbindungen mit einer Mehrstich-Zickzack-Naht ausgeführt sind.

13. Schulterbandage nach einem der Ansprüche 1 bis 12, **dadurch gekennzeichnet, dass** der Kreuzungspunkt (4) der Bänder (2,3) allseitig vernäht ist.

14. Schulterbandage nach einem der Ansprüche 1 bis 13, **dadurch gekennzeichnet, dass** die lösbaren Verbindungen Klettverbindungen sind.

15. Schulterbandage nach einem der Ansprüche 1 bis 14, **dadurch gekennzeichnet, dass** bei einer Unterarmauflage (6), die mit mindestens zwei Bändern (12-12x) versehen ist, ein unlösbarer oder lösbarer gazeartiger Teil eingelegt ist.

16. Schulterbandage nach einem der Ansprüche 1 bis 7, 13 und 14, **dadurch gekennzeichnet, dass** die Schulterbandage (1) mindestens teilweise einstückig aus einem Gestrick oder Gewirk hergestellt ist.

17. Schulterbandage nach einem der Ansprüche 1 bis 16, **dadurch gekennzeichnet, dass** nach dem Anlegen der Schlaufe (10) für den Schulterbereich (20) der Oberarm (25) und die Schlaufe (10) von einem zusätzlichen oder mit der Schlaufe (10) verbundenen und lösbaren Band (28) umschlossen ist, auf dem das Halteband (8) festgelegt ist, nach dem es zwischen dem Körper und dem Oberarm (25) durchgeführt ist oder dass der Haltegurt (8) durch eine körperseitig am Band (28) unlösbare vorgesehene Schlaufe (15) gezogen wird und anschließend rückläufig auf dem Halteband (8) fixiert ist.
